# EUROPÄISCHE PATENTSCHRIFT

(11) **EP 0 384 982 B1**
(45) Veröffentlichungstag und Bekanntmachung des Hinweises auf die Patenterteilung: **14.12.1994**
(21) Anmeldenummer: 89123898.2
(22) Anmeldetag: 23.12.1989
(51) Int. Cl.: C11D 1/06, A61K 7/075, A61K 7/50

(54) **Detergentienzusammensetzungen mit erhöhter Viskosität**
Detergent compostions with enhanced viscosity
Compositions détergentes à viscosité augmentée

(30) Priorität: 25.02.1989 DE 3905938
(43) Veröffentlichungstag der Anmeldung: 05.09.1990
(73) Patentinhaber: HÜLS AKTIENGESELLSCHAFT, 45764 Marl (DE)
(72) Erfinder: Balzer, Dieter, Dr., D-4358 Haltern (DE)

(56) Entgegenhaltungen:
- EP-A- 0 178 006
- DE-A- 2 206 284
- FR-A- 2 233 391
- SEIFEN-ÖLE-FETTE-WACHSE, Band 114, Nr. 5, März 1988, Seiten 159-166; H. MEIJER:"Die Zukunft der Tenside in der Kosmetik"

## Beschreibung

Die Erfindung betrifft wäßrige Detergentienzusammensetzungen mit erhöhter Viskosität zum Einsatz bei flüssigen Reinigungsmitteln, bzw. als Basis für Shampoonierungsmittel, Bade- und Duschgele sowie andere kosmetische Formulierungen bei denen eine erhöhte Viskosität aus Applikationsgründen gefordert ist.

In der Vergangenheit basierten solche Systeme vorwiegend auf Alkylsulfaten oder Alkylethersulfaten mit gewöhnlich niedrigen Ethoxylierungsgraden. Die Viskosität läßt sich hierbei relativ einfach durch Zugabe von Kochsalz, Ammoniumchlorid, Natriumsulfat und anderen Elektrolyten, eventuell kombiniert mit Fettsäurediethanolamiden und/oder anderen Additiven einstellen. Solche Formulierungen zeigen befriedigenden Schaum, sind preiswert, haben allerdings den gravierenden Nachteil von meist starker Hautreizung und schlechter Augenschleimhautverträglichkeit, was in Anbetracht der häufig täglich üblich gewordenen Anwendung von erheblichem Gewicht ist. Hinzu kommt in jüngerer Zeit die große Problematik möglicher toxischer Effekte aufgrund des Gehaltes an Dioxan bei Ethersulfaten und an N-Nitrosaminen bei Fettsäureamiden, weshalb möglichst stickstofffreie Formulierungen ohne die Verwendung von Ethersulfaten anzustreben sind. Die verbleibenden Alkylsulfate gehören zu den Tensiden mit geringster Haut- und Schleimhautverträglichkeit.

Die Suche nach milderen Tensiden, die diese Nachteile nicht aufweisen, ist seit einiger Zeit im Gange. Es wurden dabei zwar Tenside aufgefunden, die haut- bzw. schleimhautfreundliche Eigenschaften beinhalten, die aber andererseits den großen Nachteil besitzen, daß sie nicht bzw. nur sehr eingeschränkt elektrolytverdickbar sind (H. Meijer, Seifen-Öle-Fette-Wachse 113, 135 (1987), H. Tesmann, Parfümerie und Kosmetik 68, 630 (1987). Ausreichend hohe Viskositäten versucht man daher entweder durch die Erhöhung der Tensidkonzentration oder mittels einer sehr begrenzten Teilsubstitution des Alkylsulfats oder des Ethersulfats durch ein milderes, toxikologisch unbedenklicheres, aber leider nur schlecht verdickbares Tensid zu erzielen (US 3 038 862, H. Meijer, loc cit.). Eine Verdickung mittels wasserlöslicher Polare gilt hierbei wegen der negativen Beeinflussung der Schaumqualität sowie des Hautgefühls als wenig geeignete Alternative.

Der Zusatz kleiner Mengen carboxymethylierter Oxethylate in builderhaltigen flüssigen Reinigungsmitteln mit anionischen und eventuell nichtionischen Tensiden, die in der EP 0 178 006 beschrieben wird, führt dazu, daß die Komposition weniger empfindlich gegen den Einfluß von Elektrolyten reagiert. Eine für kosmetische Anwendungen erwünschte ausreichend hohe Viskosität ist dadurch aber offenbar nicht erreichbar.

Es bestand daher die Aufgabe, verdickbare haut- und augenschleimhautfreundliche Detergentienzusammensetzungen aufzufinden, die weitgehend frei von Alkyl- bzw. Alkylethersulfat sowie N-haltigen Tensiden sind.

Die Aufgabe wurde dadurch gelöst, daß man wäßrige Mischungen aus carboxymethylierten Oxethylaten, bei denen der Alkylrest einfach oder mehrfach ungesättigt ist, gegebenenfalls in Kombination mit carboxymethylierten Oxethylaten, bei denen der Alkylrest gesättigt ist, und Elektrolyten herstellt, wobei diese Mischungen gegebenenfalls geringe Mengen an gewöhnlichen Hilfstensiden enthalten dürfen.

Gegenstand der Erfindung ist daher eine wäßrige Detergentienzusammensetzung erhöhter Viskosität bestehend aus
a) 5 bis 30 Gew.-% carboxymethyliertem Oxethylat der Formel (I)

   R-O-(C₂H₄O)ₙ CH₂ COOM (I),

   wobei R einen einfach oder mehrfach ungesättigten, verzweigten oder unverzweigten aliphatischen Rest mit 10 bis 22 C-Atomen,
   n 1 bis 10 und
   M ein Alkali-, Erdalkali-, Ammonium- oder Alkylammoniumion bedeuten,
b) 0 bis 20 Gew.-% carboxymethyliertem Oxethylat der Formel (II)

   R'-O-(C₂H₄O)ₘ CH₂ COOM' (II),

   wobei R' einen gesättigten, verzweigten oder unverzweigten aliphatischen Rest mit 10 bis 22 C-Atomen, oder einen alkylaromatischen Rest mit 8 bis 18 C-Atomen in der Alkylgruppe
   m 1 bis 10 und
   M′ ein Alkali-, Erdalkali-, Ammonium- oder Alkylammoniumion bedeuten,
c) 0,2 bis 6 Gew.-% Elektrolyt und
d) gegebenenfalls Hilfstensiden und Wasser ad 100 Gew.-%.

Die schon seit langem bekannte Substanzgruppe (US-PS 2 183 853) der Formel

R-O-(C₂H₄O)ₙ CH₂ COOM,

wobei R einen aliphatischen oder alkylaromatischen Rest, n der Ethoxylierungsgrad und M ein Metallion bedeuten, lassen sich nach DE-OS 24 18 444 bzw. EP-A 0 106 018 aus den entsprechenden Oxethylaten durch Umsetzung mit Chloressigsäure bzw. nach EP-A 0 018 681 oder DE-OS 28 16 127 durch Oxydation herstellen. Aufgrund ihrer hohen Oberflächenaktivität sowie ihrer sehr guten Haut- bzw. Augenschleimhautverträglichkeit finden sie seit einigen Jahren besonders in kosmetischen Formulierungen sowie auch in Reinigungssystemen Anwendung (N.A.I. Paassen, Seifen-Öle-Fette-Wachse 109, 353 (1983). Allerdings weisen die carboxymethylierten Oxethylate bei aller attraktiven Hautfreundlichkeit den Nachteil auf, nur schwierig verdickbar zu sein (Hamke Meijer, Seifen-Fette-Öle-Wachse 114, 159 (1988). Entsprechend der EP-A 0 176 151 sind die carboxymethylierten Oxethylate durch Elektrolyte überhaupt nicht zu verdicken.

Carboxymethylierte Oxethylate üblicher gesättigter C₁₂/C₁₄-Fettalkohole lassen sich de facto kaum durch NaCl oder andere übliche Elektrolyte verdicken. Geht man zu längeren Kohlenwasserstoffketten über, so nimmt zwar die Verdickbarkeit ein wenig zu, aber es kommt zur Bildung von sehr trüben Lösungen. So ist ein klares zähflüssiges (≧ 1 000 mPa · s), elektrolytverdicktes, carboxymethyliertes Oxethylat eines gesättigten Fettalkohols z.B. in einer wäßrigen Lösung, die weniger als 10 % Tensid enthält, nicht herstellbar.

Es wurde nun überraschend gefunden, daß carboxymethylierte Oxethylate basierend auf ungesättigten Alkoholen oder diese im Gemisch mit carboxymethylierten Oxethylaten basierend auf gesättigten Alkoholen hochviskose, gelartige, aber völlig klare wäßrige Systeme mit Elektrolyten als Verdickungsmittel bilden, obwohl der Gehalt an waschaktiver Substanz nur 10 Gew.-% und der Elektrolytgehalt nur 1 - 3 Gew.-% betragen.

### Carboxymethylierte Oxethylate

Erfindungsgemäß eingesetzte carboxymethylierte Oxethylate entsprechen der Formel (I)

R-O-(C₂H₄O)ₙ CH₂ COOM (I),

in der R einen linearen oder verzweigten, einfach oder mehrfach ungesättigten Kohlenwasserstoffrest mit 10 bis 22, vorzugsweise 12 bis 20 Kohlenstoffatomen, n 1 bis 10, vorzugsweise 2 bis 8, und M Metallionen wie Natrium-, Kalium- oder Erdalkali- bzw. Ammonium- oder Alkylammoniumion, vorzugsweise Natriumion, bedeuten.

Eine gegebenenfalls weitere, aber nur in Kombination mit Verbindungen der Formel (I), erfindungsgemäß eingesetzte Gruppe der carboxymethylierten Oxethylate entsprechen der Formel (II)

R′-O-(C₂H₄O)ₘ CH₂ COOM′ (II),

in der R′ einen gesättigten, verzweigten oder unverzweigten aliphatischen Rest mit 10 bis 22, vorzugsweise 12 bis 18 C-Atomen, oder einen alkylaromatischen Rest mit 8 bis 18, vorzugsweise 8 bis 14 C-Atomen in der Alkylgruppe, m 1 bis 10, vorzugsweise 3 bis 8 und M′ Metallionen wie Natrium-, Kalium-, Erdalkali- bzw. Ammonium- oder Alkylammoniumion, vorzugweise Natriumion, bedeuten.

Die Herstellung dieser Verbindungen gelingt z.B. durch Umsetzung der Fettalkoholoxethylate mit Chloressigsäure in Gegenwart von Basen wie NaOH. In Abhängigkeit vom Oxethylat/Chloressigsäureverhältnis ist die Umsetzung mehr oder weniger quantitativ, so daß das erfindungsgemäß eingesetzte carboxymethylierte Oxethylat häufig ein Gemisch von Ausgangsoxethylat und Umsetzungsprodukt ist. Abhängig von der Verwendung kann das mitentstehende Salz in vielen Fällen im Produkt verbleiben. Geeignet, insbesondere auch für kosmetische Anwendungen, sind carboxymethylierte Oxethylate auf der Basis Oleylalkohol, Elaidylalkohol, Linoleylalkohol, Linolenylalkohol, Gadoleylalkohol, Arachidonalkohol, Erucaalkohol oder Ricinolalkohol etc. bzw. deren Gemische.

Werden diese carboxymethylierten Oxethylate mit ungesättigten Kohlenwasserstoffresten gemeinsam mit solchen mit gesättigten Kohlenwasserstoffresten eingesetzt, so sollte das Verhältnis 10 : 1 bis 1 : 4, vorzugsweise 3 : 1 bis 1 : 2 betragen. Die Konzentration in wäßrigen Lösungen betragen für Verbindungen mit ungesättigtem Rest 5 bis 30, vorzugsweise 7 bis 20 Gew.-% bzw. für Verbindungen mit gesättigten Resten 0 bis 20, vorzugsweise 2 bis 10 Gew.-%.

### Elektrolyte

Erfindungsgemäß eingesetzte Elektrolyte sind übliche Elektrolyt-Verdicker, wie z.B. NaCl, NH₄Cl, Na₂SO₄ oder MgSO₄ etc. Die Konzentrationen betragen 0,2 bis 6 Gew.-%, vorzugsweise 0,5 bis 5 Gew.-%.

### Hilfstenside

Die erfindungsgemäß eingesetzten carboxymethylierten Oxethylate sind im Hinblick auf ihre Anwendung gewöhnlich ausreichend schäumend. Sind höhere Schaumnoten erwünscht, so empfiehlt sich die Zugabe kleinerer Mengen stark schäumender Hilfstenside, wie z.B. organische Sulfate, Sulfonate, Aminoxide, Amphotenside oder deren Gemische. Die Mengenverhältnisse dieser Hilfstenside zu den carboxymethylierten Oxethylaten betragen 3 : 7 bis 1 : 9. In manchen, aber im Hinblick auf ihre Umweltproblematik seltenen Fällen kann es nützlich sein, zur Viskositätsregulation Fettsäureamide in kleinen Mengen mitzuverwenden.

Als weitere Additive in Abhängigkeit vom Einsatzgebiet kommen kleinere Mengen an Polymeren, wie Polyethylenoxid, Chelatbildner, Konservierungsmittel, Duftstoffe etc. in Frage.

Mit den folgenden Beispielen soll die effektive Verdickbarkeit der erfindungsgemäßen carboxymethylierten Oxethylate durch Elektrolyte aufgezeigt werden. Sie wird nachgewiesen durch Viskositätsmessungen mit einem Rotationsviskosimeter (Haake RV 20) bei 25 °C unter definierten Scherraten. Bei stark strukturviskosen Zubereitungen werden die mittleren Viskositäten bei Scherraten zwischen 3 und 10 sec⁻¹ mitgeteilt, bei Bedingungen also, die etwa dem Bewegungsvorgang beim Ausfluß einer Flüssigkeit aus einer Plastikflasche mit mittlerer Öffnung entsprechen. Der Gehalt an waschaktiver Substanz bei allen Beispielen beträgt 10 %.

### Beispiel 1 (erfindungsgemäß)

In einem Glasgefäß werden carboxymethyliertes Ocenol-80/85*-Oxethylat-Natriumsalz mit 3 mol Ethylenoxid/mol (Carboxymethylierungsgrad 88 %, Produkt enthält 9,8% NaCl) und weiteres NaCl in Wasser gelöst und die Viskosität der Lösungen nach 48 h Stehen gemessen.
* Gemisch Oleylalkohol-Cetylalkohol der Firma Henkel

| Gesamt-NaCl (%) | 1 | 1,5 | 2,5 | 3,5 |
|---|---|---|---|---|
| Aussehen der Lösung nach 48 h | klar | klar | trüb | 2 Phasen |
| η 25 °C (mPa·s) | 880 | 2 900 | 7 000 | - |

Die Ergebnisse zeigen die effektive Verdickbarkeit der erfindungsgemäßen carboxymethylierten Oxethylate.

### Beispiel 2 (erfindungsgemäß)

Carboxymethyliertes Ocenol-80/85-Oxethylat, mit 4 mol Ethylenoxid/mol (Carboxymethylierungsgrad 90 %, Produkt enthält 10,1 % NaCl) und weiteres NaCl werden in Wasser gelöst und die Viskosität der klaren Lösung nach etwa 48 h Stehen gemessen.

| Gesamt-NaCl (%) | 1,0 | 1,5 | 2,0 | 2,5 | 3,0 | 3,5 | 4 |
|---|---|---|---|---|---|---|---|
| η 25 °C (mPa·s) | 3 | 6 | 8 | 45 | 700 | 1 500 | 2 800 |

### Beispiel 3 (Vergleichsbeispiel)

Carboxymethyliertes Alfol-12-14-Oxethylat mit 4,5 mol Ethylenoxid/mol (Carboxymethylierungsgrad 80 %, Produkt enthält 8,5 % NaCl) und weiteres NaCl werden in Wasser gelöst und die Viskosität der klaren Lösungen bestimmt.

| Gesamt-NaCl(%) | 2 | 4 | 6 | 8 | 10 | 12 |
|---|---|---|---|---|---|---|
| 25 °C (mPa·s) | 1 | 1,3 | 2,3 | 6 | 25 | 70 |

Die Ergebnisse mit diesem carboxymethylierten Oxethylat mit gesättigter, aliphatischer Gruppe zeigen, daß hier keine für die praktische Anwendung ausreichende Verdickbarkeit erzielt wird.

### Beispiel 4 (Vergleichsbeispiel)

Es wird versucht, hydriertes carboxymethyliertes Talgfett-C₁₆C₁₈-Oxethylat mit 5 mol Ethylenoxid/mol (Carboxymethylierungsgrad 91 %, Produkt enthält 9,2 % NaCl) zusammen mit weiterem NaCl in Wasser zu lösen. Die Flüssigkeiten waren bei allen NaCl-Konzentrationen trüb mit deutlicher Tendenz zur Phasentrennung, so daß keine praktische Anwendung gegeben ist.

| Gesamt-NaCl (%) | 1 | 2 | 3 | 4 |
|---|---|---|---|---|
| Aussehen der Lösung | trüb | trüb (Perlglanz) | 2-phasig | |
| 25 °C (mPa·s) | 900 | 700 | 450 | - |

### Beispiel 5 (erfindungsgemäß)

Carboxymethyliertes Ocenol-92/96*-Oxethylat mit 4 mol Ethylenoxid/mol (Carboxymethylierungsgrad 91 %, Produkt enthält 10,1 % NaCl) wurde zusammen mit weiterem NaCl in Wasser gelöst und nach längerem Stehen hinsichtlich ihrer Viskosität vermessen. Die über den gesamten NaCl-Bereich klaren Lösungen besitzen bei NaCl-Konzentration ≧ 3 % gelartige Konsistenz.
* Oleylalkohol der Fa. Henkel

| Gesamt-NaCl (%) | 1 | 2 | 3 | 4 | 6 |
|---|---|---|---|---|---|
| η 25 °C (mPa·s) | 5 | 25 | 1 700 | 6 500 | 7 000 |

### Beispiel 6 (erfindungsgemäß)

Carboxymethyliertes Ocenol-110/130*-Oxethylat mit 4 mol Ethylenoxid/mol (Carboxymethylierungsgrad 89 %, Produkt enthält 10,0 % NaCl) und weiteres NaCl werden zusammen in Wasser gelöst und die Viskosität der klaren Lösungen gemessen.
* Oleyl-Linoleyl-Alkohol der Fa. Henkel

| Gesamt-NaCl (%) | 1 | 1,5 | 2 | 3 | 4 |
|---|---|---|---|---|---|
| η 25 °C (mPa·s) | 2 | 8 | 130 | 2 900 | 4 800 |

### Beispiel 7 (erfindungsgemäß)

Carboxymethyliertes Ocenol-92/96-Oxethylat mit 3,5 mal Ethylenoxid/mol (Umsetzungsgrad 91 %, Produkt enthält 10 % NaCl) und NH₄Cl werden zusammen in Wasser gelöst und die Viskosität der klaren, z. T. stark geligen Lösungen gemessen.

| Gesamt-NH₄Cl (%) | - | 0,5 | 1 | 2 | 3 |
|---|---|---|---|---|---|
| η 25 °C (mPa·s) | 12 | 948 | 4 030 | 6 200 | 9 700 |

### Beispiel 8 (erfindungsgemäβ)

Carboxymethyliertes Ocenol-80/85-Oxethylat mit 4,5 mol Ethylenoxid/mol (Umsetzungsgrad 90 %, Produkt enthält 9,8 % NaCl) und carboxymethyliertes C₁₆C₁₈ Talgfettalkohol**-Oxethylat mit 5 mal Ethylenoxid/mal (Umsetzungsgrad 91 %, Produkt enthält 9,2 % NaCl) werden im Massenverhältnis 3 : 1 zusammen mit NH₄Cl in Nasser gelöst. Die Viskositätsmessung der klaren Lösungen ergibt folgende Abhängigkeit:
** ca. 29 % C₁₆H₃₃OH, 68 % C₁₈H₃₇OH

| NH₄Cl (%) | - | 2 | 3 | 4 | 5 |
|---|---|---|---|---|---|
| η 25 °C (mPa·s) | 4 | 27 | 410 | 2 750 | 4 300 |

### Beispiel 9 (erfindungsgemäß)

Carboxymethyliertes Ocenol-92/96-Oxethylat mit 3 mol Ethylenoxid/mol, und C₁₄/C₁₆-Olefinsulfonat im Massenverhältnis 4 : 1 wurden gemeinsam mit zusätzlichem NaCl in Wasser gelöst. Nach 48 h Stehen der klaren Lösungen wurde die Viskosität bestimmt:

| Gesamt-NaCl (%) | 1 | 2 | 3 |
|---|---|---|---|
| η 25 °C (mPa·s) | 16 | 860 | 10 100 |

## Patentansprüche

1. Wäßrige Detergentienzusammensetzung erhöhter Viskosität bestehend aus
a) 5 bis 30 Gew.-% carboxymethyliertem Oxethylat der Formel (I)
R-O-(C₂H₄O)ₙ CH₂ COOM (I),
wobei R einen einfach oder mehrfach ungesättigter verzweigter oder unverzweigter aliphatischer Rest mit 10 bis 22 C-Atomen,
n 1 bis 10 und
M ein Alkali-, Erdalkali-, Ammonium- oder Alkylammoniumion bedeuten,
b) 0 bis 20 Gew.-% carboxymethyliertem Oxethylat der Formel (II)
R′-O-(C₂H₄O)ₘ CH₂ COOM′ (II),
wobei R′ einen gesättigter, verzweigter oder unverzweigter, aliphatischer Rest mit 10 bis 22 C-Atomen, oder einen alkylaromatischen Rest mit 8 bis 18 C-Atomen in der Alkylgruppe,
m 1 bis 10 und
M′ ein Alkali-, Erdalkali- oder Ammonium- oder Alkylammoniumion bedeuten,
c) 0,2 bis 6 Gew.-% Elektrolyt und
d) gegebenenfalls Hilfstensiden und Wasser ad 100 Gew.-%

2. Wäßrige Detergentienzusammensetzung erhöhter Viskosität nach Anspruch 1,
dadurch gekennzeichnet,
daß carboxymethyliertes Oxethylat der Formel (I), in der R = Oleyl-, Linoleyl, Linolenyl- und Ricinol oder Gemische davon bedeutet, verwendet wird.

3. Wäßrige Detergentienzusammensetzung erhöhter Viskosität nach Anspruch 1,
dadurch gekennzeichnet,
daß carboxymethyliertes Oxethylat der Formel (II), in der R' einen gesättigten, verzweigten oder unverzweigten aliphatischen Rest mit 12 bis 18 C-Atomen, oder einen alkylaromatischen Rest mit 8 bis 18 C-Atomen in der Alkylgruppe bedeutet, verwendet wird.

4. Wäßrige Detergentienzusammensetzung erhöhter Viskosität nach Anspruch 1,
dadurch gekennzeichnet,
daß als Elektrolyte NaCl und/oder NH₄Cl verwendet werden.

5. Wäßrige Detergentienzusammensetzung erhöhter Viskosität nach Anspruch 1,
dadurch gekennzeichnet,
daß die Menge des carboxymethylierten Oxethylats der Formel (I) 7 bis 20 Gew.-% beträgt.

6. Wäßrige Detergentienzusammensetzung erhöhter Viskosität nach Anspruch 1,
dadurch gekennzeichnet,
daß die Menge der Elektrolyte 0,5 bis 5 Gew.-% beträgt.

7. Wäßrige Detergentienzusammensetzung erhöhter Viskosität nach Anspruch 1,
dadurch gekennzeichnet,
daß das Verhältnis der carboxymethylierten Oxethylate der Formel (I) : (II) von 10 : 1 bis 1 : 4 beträgt.

8. Verwendung der wäßrigen Detergentienzusammensetzung nach Anspruch 1, als Basis für Shampoonierungsmittel, Bade- und Duschgele bzw. Reinigungsmittel, wobei die Viskosität der wäßrigen Detergentienzusammensetzung mindestens 1 000 mPa·s beträgt.

## Claims

1. An aqueous detergent composition of increased viscosity composed of
a) 5 to 30% by weight of carboxymethylated oxyethylate of the formula (I)
R-O-(C₂H₄O)ₙCH₂COOM (I),
where R is a mono- or poly-unsaturated, branched or unbranched, aliphatic radical having 10 to 22 C atoms,
n is 1 to 10 and
M is an alkali metal, alkaline earth metal, ammonium or alkylammonium ion,
b) 0 to 20% by weight of carboxymethylated oxyethylate of the formula (II)
R'-O-(C₂H₄O)ₘCH₂COOM' (II),
where R' is a saturated, branched or unbranched, aliphatic radical having 10 to 22 C atoms, or an alkylaromatic radical having 8 to 18 C atoms in the alkyl group,
m is 1 to 10 and
M' is an alkali metal, alkaline earth metal or ammonium or alkylammonium ion,
c) 0.2 to 6% by weight of electrolyte, and
d) if desired auxiliary surfactants, and water to 100% by weight.

2. An aqueous detergent composition of increased viscosity according to claim 1, characterised in that carboxymethylated oxyethylate of the formula (I) in which R is oleyl, linoleyl, linolenyl or ricinoleyl or mixtures thereof is used.

3. An aqueous detergent composition of increased viscosity according to claim 1, characterised in that carboxymethylated oxyethylate of the formula (II) in which R' is a saturated, branched or unbranched, aliphatic radical having 12 to 18 C atoms or an alkylaromatic radical having 8 to 18 C atoms in the alkyl group is used.

4. An aqueous detergent composition of increased viscosity according to claim 1, characterised in that NaCl and/or NH₄Cl are used as electrolytes.

5. An aqueous detergent composition of increased viscosity according to claim 1, characterised in that the amount of carboxymethylated oxyethylate of the formula (I) is 7 to 20% by weight.

6. An aqueous detergent composition of increased viscosity according to claim 1, characterised in that the amount of electrolytes is 0.5 to 5% by weight.

7. An aqueous detergent composition of increased viscosity according to claim 1, characterised in that the ratio of the carboxymethylated oxyethylates of the formula (I):(II) is from 10:1 to 1:4.

8. The use of the aqueous detergent composition according to claim 1 as a basis for shampooing agents, bath and shower gels or cleaning agents, the viscosity of the aqueous detergent composition being at least 1,000 mPa·s.

## Revendications

1. Composition aqueuse de détergents, d'une viscosité élevée, constituée par :
a) 5 à 30 % en poids d'un oxéthylat carboxy-méthylé de la formule (I)
R-O-(C₂H₄O)ₙ CH₂ COOM (I),
dans laquelle R représente un radical aliphatique à insaturation simple ou multiple, qui est ramifié ou non ramifié et comporte de 10 à 22 atomes de carbone,
n a une valeur de 1 à 10, et
M est un ion alcalin, alcalino-terreux, ammonium ou alkyl-ammonium,
b) 0 à 20 % en poids d'un oxéthylat carboxy-méthylé de la formule (II)
R'-O-(C₂H₄O)ₘ CH₂ COOM' (II),
dans laquelle R' représente un radical aliphatique saturé, ramifié ou non ramifié, comportant de 10 à 22 atomes de carbone, ou un radical alkyl-aromatique comportant de 8 à 18 atomes de carbone dans le groupe alkyle,
m a une valeur de 1 à 10, et
M' représente un ion alcalin, alcalino-terreux ou ammonium, ou un ion alkyl-ammonium,
c) 0,2 à 6 % en poids d'un électrolyte, et
d) le cas échéant des tensio-actifs auxiliaires et de l'eau jusqu'à 100 % en poids.

2. Composition aqueuse de détergents, d'une viscosité élevée selon la revendication 1,
caractérisée en ce que l'on utilise un oxéthylat carboxyméthylé de la formule (I), dans laquelle R représente un radical oléyle, linoléyle, linolényle et ricinol, ou des mélanges ces radicaux.

3. Composition aqueuse de détergents, d'une viscosité élevée selon la revendication 1,
caractérisée en ce que l'on utilise un oxéthylat carboxyméthylé de la formule (II), dans laquelle R' représente un radical aliphatique saturé, ramifié ou non ramifié, qui comporte de 12 à 18 atomes de carbone, ou bien un radical alkyl-aromatique comportant de 8 à 18 atomes de carbone dans le groupe alkyle.

4. Composition aqueuse de détergents, d'une viscosité élevée selon la revendication 1,
caractérisée en ce que l'on utilise comme électrolyte NaCl et/ou NH₄Cl.

5. Composition aqueuse de détergents, d'une viscosité élevée selon la revendication 1,
caractérisée en ce que la quantité de l'oxéthylat carboxyméthylé de la formule (I) est de 7 à 20 % en poids.

6. Composition aqueuse de détergents, d'une viscosité élevée selon la revendication 1,
caractérisée en ce que la quantité de l' électrolyte est de 0,5 à 5 % en poids.

7. Composition aqueuse de détergents, d'une viscosité élevée selon la revendication 1,
caractérisée en ce que la proportion des oxéthylats carboxy-méthylés des formules (I) : (II) est de 10 : 1 à 1 : 4.

8. L'utilisation de la composition aqueuse de détergents, sel on la revendication 1, comme base pour des schampooings, des gels pour bain et des gels pour douche, des agents de nettoyage, la viscosité de la composition aqueuse de détergents atteignant au moins 1 000 mPa.s.
